(19) Europäisches Patentamt
European Patent Office
Office européen des brevets

(11) Veröffentlichungsnummer: **0 159 644**
**B1**

# (12) EUROPÄISCHE PATENTSCHRIFT

(45) Veröffentlichungstag der Patentschrift: **06.04.88**

(51) Int. Cl.⁴: **C 07 D 207/48**, A 01 N 43/36

(21) Anmeldenummer: **85104530.2**

(22) Anmeldetag: **15.04.85**

## (54) N-(Dichlorfluormethylthio)-3,4-dimethylmaleinimid, ein Verfahren zu seiner Herstellung und seine Verwendung.

(30) Priorität: **26.04.84 DE 3415532**

(43) Veröffentlichungstag der Anmeldung: **30.10.85 Patentblatt 85/44**

(45) Bekanntmachung des Hinweises auf die Patenterteilung: **06.04.88 Patentblatt 88/14**

(84) Benannte Vertragsstaaten: **BE CH DE FR GB IT LI NL SE**

(56) Entgegenhaltungen:
**EP-A-0 111 452**
**DE-A-3 203 057**
**GB-A-927 834**
**US-A-3 499 030**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Kühle, Engelbert, Dr., von-Bodelschwingh- Strasse 42, D-5060 Bergisch-Gladbach 2 (DE)**
Erfinder: **Paulus, Wilfried, Dr., Deswatinesstrasse 90, D-4150 Krefeld 1 (DE)**
Erfinder: **Genth, Hermann, Dr., Am Heckerhof 60, D-4150 Krefeld 1 (DE)**

EP 0 159 644 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft das neue N-(Dichlorfluormethylthio) -3,4-dimethylmaleinimid, ein Verfahren zu seiner Herstellung und seine Verwendung als mikrobizides Mittel.

Die Verwendung von N-(Trihalogenmethylthio)-Verbindungen zum Schutz technischer Materialien gegen mikrobiellen Abbau ist bekannt (US 2 563 770; Journ. Agr. Food Chem. 14, 365 (1966); Fette, Seifen, Anstrichmittel 68, 272 (1966), GB-A-927 834, US-A-3 499 030 und DE-A-3 203 057). Sie befriedigen jedoch nicht immer, da sie besonders in einigen Anstrich- und Imprägniermitteln schlecht löslich sind.

Es wurde das neue N-(Dichlorfluormethylthio)-3,4-di-methylmaleinimid gefunden.

Das neue N-(Dichlorfluormethylthio)-3,4-dimethylmalein-imid hat hervorragende mikrobizide Eigenschaften und zeigt eine gute Löslichkeit im besonderen in Anstrich- und Imprägniermitteln.

Es wurde auch ein Verfahren zur Herstellung des neuen N-(Dichlorfluormethylthio)-3,4-dimethylmaleinimids gefunden, das dadurch gekennzeichnet ist, daß man 3,4-Di-methylmaleinimid mit Dichlorfluormethansulfenylchlorid in Gegenwart eines säurebindenden Mittels in Lösung umsetzt.

Das erfindungsgemäße Verfahren kann durch die folgende Reaktionsgleichung erläutert werden:

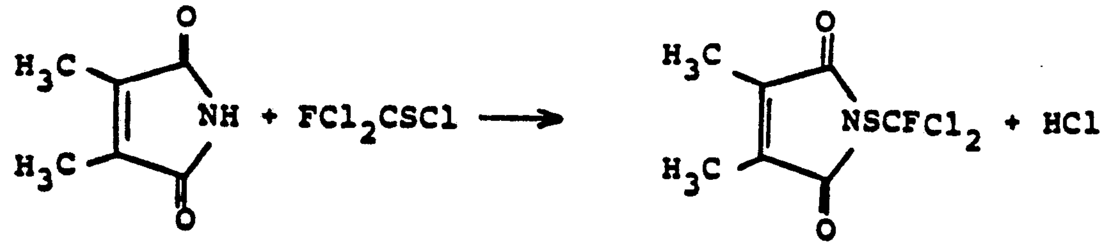

Das 3,4-Dimethylmaleinimid ist aus Beilsteins Handbuch der organischen Chemie, Band 21, Seite 412, bekannt.

Dichlorfluormethansulfenylchlorid wird in Angew. Chem. 76, 807 (1964) beschrieben.

Säurebindende Mittel für das erfindungsgemäße Verfahren können beispielsweise Natriumhydroxid, Natriumcarbonat, Triethylamin oder Pyridin sein.

Lösungsmittel für das erfindungsgemäße Verfahren können beispielsweise Kohlenwasserstoffe wie Toluol, Chlorkohlenwasserstoffe wie Chlorbenzol, Ether wie Dioxan, oder Wasser sein. Das erfindungsgemäße Verfahren wird im allgemeinen im Temperaturbereich von 0 bis 100° C, bevorzugt von 20 bis 50° C, durchgefürt.

Das erfindungsgemäße Verfahren wird im allgemeinen bei Normaldruck durchgeführt. Es ist jedoch auch möglich, das Verfahren bei einem Über- oder Unterdruck (beispielsweise im Druckbereich von 0,5 bis 1,5 bar) durchzuführen.

Das erfindungsgemäße N-(Dichlorfluormethylthio)-3,4-dimethylmaleinimid kann als Wirkstoff zur Bekämpfung von Mikroorganismen, im besonderen zum Schutz technischer Materialien und im Pflanzenschutz verwendet werden.

Technische Materialien sind erfindungsgemäß nicht lebende Materialien, die für die Verwendung in der Technik zubereitet worden sind. Beispielsweise können technische Materialien, die durch den erfindungsgemäßen Wirkstoff vor einer mikrobiellen Veränderung oder Zerstörung geschützt werden sollen, Klebstoffe, Leime, Papiere und Kartone, Textilien, Leder, Holz, Anstrichmittel und Kunststoffe, Kühlschmiermittel und andere Materialien sein, diedurch Mikroorganismen zersetzt werden können. Im Rahmen der zu schützenden Materialien seien auch Teile von Produktionsanlagen, beispielsweise Kühlwasserkreisläufe genannt, die durch Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt. Im besonderen sei der Schutz von Vorrichtungen und/oder Gerätschaften aus Holz genannt.

Mikroorganismen, die einen Abbau oder eine Veränderung der technischen Materialien bewirken können, sind beispielsweise Bakterien, Pilze, Hefen, Algen und Schleimorganismen. Vorzugsweise wirkt der erfindungsgemäße Wirkstoff gegen Schimmelpilze, holzverfärbende Pilze und holzzerstörende Pilze (Basidiomyceten) sowie gegen Schleimorganismen.

Es seien beispielsweise Mikroorganismen der folgenden Gattungen genannt:.

Alternaria, wie Alternaria tenuis,
Aspergillus, wie Aspergillus niger,
Aureobasidium, wie Aureobasidium pullulans,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Sclerophoma, wie Sclerophoma pityophila,
Staphylococcus, wie Staphylococcus aureus.

Je nach ihrem Anwendungsgebiet kann der erfindungsgemäße Wirkstoff in die üblichen Formulierungen überführt werden, wie Lösungen, Emulsionen, Suspensionen, Pulver, Pasten und Granulate.

Diese können in an sich bekannter Weise hergestellt werden, z. B. durch Vermischen des Wirkstoffes mit einem Streckmittel das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei beispielsweise im Falle der Benutzung von Wasserstreckmitteln gegebenenfalls organische Lösungsmittel wie Alkohole als Hilfsmittel verwendet werden können.

Organische Lösungsmittel für den erfindungsgemäßen Wirkstoff können beispielsweise Alkohole, wie niedere aliphatische Alkohole, vorzugsweise Ethanol oder Isopropanol, oder Benzylalkohol, Ketone, vorzugsweise Aceton oder Methylethylketon, flüssige Kohlenwasserstoffe, vorzugsweise Benzinfraktionen, oder chlorierte Kohlenwasserstoffe, vorzugsweise 1,2-Dichlorethan sein.

Die mikrobiziden Mittel enthalten den Wirkstoff im allgemeinen in einer Menge von 1 bis 95 %, bevorzugt von 10 bis 75 %.

Die Anwendungskonzentrationen des erfindungsgemäßen Wirkstoffs richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material. Der erfindungsgemäße Wirkstoff kann auch in Mischung mit anderen an sich bekannten Wirkstoffen vorliegen. Beispielsweise seien die folgenden Wirkstoffe genannt: Benzylalkoholmono(poly)hemiformal Benzimidazolyl-methylcarbamate, Tetramethylthiuramdisulfid, Zinksalze von Dialkylthiocarbamaten, 2,4,5,6-Tetrachlorisophthalonitril, Thiazolylbenzimidazol, Mercaptobenzthiazol, Trialkylzinnverbindungen, Methylenbisthiocyanat und Phenolderivate, wie 2-Phenylphenol, (2,2'-Dihydroxy-5,5'-dichlor)-diphenylmethan und 3-Methyl-4-chlorphenol.

Der erfindungsgemäße Wirkstoff ist auch für den Einsatz in Pflanzenschutzmittel geeignet. Die gute Pflanzenverträglichkeit des Wirkstoffs in den zur Bekämpfung von Pflanzenkrankheiten notwendigen Konzentrationen erlaubt eine Behandlung von oberirdischen Pflanzenteilen, von Pflanzen- und Saatgut und des Bodens.

**Herstellungsbeispiel**

**Beispiel 1**

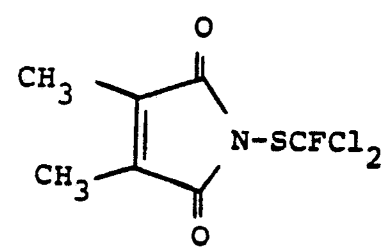

10,1 g (0,08 Mol) Dimethylmaleinimid werden unter Zusatz von 15 g (0,089 Mol) Dichlorfluormethansulfenylchlorid in 100 ml Toluol gelöst. Bei Raumtemperatur tropft man hierzu 9,4 g (0,093 Mol) Triethylamin und läßt die Temperatur bis etwa 60°C ansteigen. Man wäscht die Reaktionslösung mit Wasser, trocknet die Toluolphase, engt die Lösung im Vakuum ein und kristallisiert den Rückstand aus Waschbenzin um. Schmelzpunkt 47 bis 49°C. Ausbeute 13,5 g.

**Anwendungsbeispiele**

**Beispiel 1**

Zum Nachweis der Wirksamkeit gegen Pilze werden die minimalen Hemm-Konzentrationen (MHK) von N-(Dichlorfluormethylthio)-3,4-dimethylmaleinimid bestimmt:

Ein Agar, der aus Bierwürze und Pepton hergestellt wird, wird mit N-(Dichlorfluormethylthio)-3,4-dimethylmaleinimid in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2-wöchiger Lagerung bei 28°C und 60 bis 70 % relativer Luftfeuchtigkeit wird die MHK bestimmt. MHK ist die niedrigste Konzentration an Wirkstoff, bei der keinerlei Bewuchs durch die verwendete Mikrobenart erfolgt, sie ist in der folgenden Tabelle angegeben.

MHK-Werte in mg/l bei der Einwirkung von N-(Dichlorfluormethylthio) -3,4-dimethylmaleinimid auf Pilze

| | |
|---|---|
| Alternaria tenuis | 0,75 |
| Aspergillus niger | 50 |
| Aureobasidium pullulans | 0,5 |
| Chaetomium globosum | 50 |
| Coniophora puteana | 0,75 |
| Lentinus tigrinus | 1,5 |
| Penicillium glaucum | 5 |
| Polyporus versicolor | 7,5 |
| Sclerophoma pityophila | 1 |
| Trichoderma viride | 100 |

**Beispiel 2** (Wirkung gegen Schleimorganismen)

N-(Dichlorfluormethylthio)-3,4-dimethylmaleinimid wird in Konzentrationen von jeweils 0,1 bis 100 mg/l in Allens Nährlösung (Arch. Mikrobiol. 17, 34 bis 53 (1952)), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, in wenig Aceton gelöst, zur Anwendung gebracht. Kurz vorher werden die Nährlösungen mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3-wöchiger Kultur bei Raumtemperatur noch völlig klar, d.h. die in wirkstofffreien Nährlösungen nach 3 bis 4 Tagen bemerkbare starke Vermehrung der Mikroben und Schleimbildung unterbleibt.

Für N-(Dichlorfluormethylthio)-3,4-dimethylmaleinimid läßt sich auf diese Weise folgende MHK ermitteln: 7,5 mg/l.

**Beispiel 3**

Prüfung N-(Dichlorfluormethylthio)-3,4-dimethylmaleinimid als Anstrichfungizid.

Die Prüfung wird in Anlehnung an den Report 219 der Defense Standards Laboratories Maribyrnong/Australien wie folgt durchgeführt: Ein glatter Karton wird beidseitig mit dem zu prüfenden Anstrichmittel bestrichen und 8 Tage bei Raumtemperatur getrocknet. Zur Alterung wird ein Teil des Anstriches 24 Stunden in fließendem Wasser von 24°C gewässert oder 8 Tage mit Frischluft von 40 bis 60°C belüftet oder 110 Stunden einem trockenen Xenon-Test ausgesetzt. Von den so vorbereiteten Proben werden Abschnitte von 5 x 5 cm einzeln in Petrischalen auf einen Traubenzucker-Nährboden aufgelegt und mit einer Sporensuspension der nachstehenden Pilze kontaminiert: Aspergillus niger, Aureobasidium pullulans, Alternaria tenuis, Penicillium citrinum, Stachybotrys atra, Paecilamyces varioti, Cladosporium herbarum, Aspergillus ustus und Aspergillus flavus.

Die kontaminierten Schalen werden bei 28 bis 30°C und 90 bis 95 % relativer Luftfeuchtigkeit gelagert und nach 3 Wochen ausgewertet. Anstriche gelten als schimmelfest, wenn die Proben nach diesen Tests pilzfrei bleiben.

Nach der oben angegebenen Testmethode wird eine handelsübliche Lackfarbe auf Alkydharz-Basis auf Schimmelfestigkeit geprüft.

Nach dem Test ergibt sich die folgende Beurteilung:

Anstriche der Lackfarbe, die 0,8 % N-(Dichlorfluormethylthio) -3,4-dimethylmaleinimid bezogen auf Filmgewicht, enthalten, sind schimmelfest, auch wenn sie vor der mikrobiologischen Prüfung den obengenannten Alterungsprozessen ausgesetzt wurden.

**Patentansprüche**

1. N-(Dichlorfluormethylthio)-3,4-dimethylmaleinimid.
2. Verfahren zur Herstellung von N-(Dichlorfluormethylthio) -3,4-dimethylmaleinimid, dadurch gekennzeichnet, daß man 3,4-Dimethylmaleinimid mit Dichlorfluormethansulfenylchlorid in Gegenwart eines säurebindenden Mittels in Lösung umsetzt.
3. Mikrobizides Mittel, enthaltend als Wirkstoff N-(Dichlorfluormethylthio)-3,4-dimethylmaleinimid.
4. Mikrobizides Mittel nach Anspruch 3, enthaltend 1 bis 95 Gew.-% des Wirkstoffs.
5. Verwendung von mikrobiziden Mitteln nach Anspruch 3, zum Schutz technischer Materialien vor mikrobieller Veränderung und Zerstörung.

6. Verwendung nach Anspruch 5, dadurch gekennzeichnet, daß man 0,001 bis 5 Gew.-% des Wirkstoffs bezogen auf das zu schützende Material einsetzt.

## Claims

1. N-(Dichlorofluoromethylthio)-3,4-dimethylmaleimide.
2. Process for the preparation of N-(dichlorofluoromethylthio) -3,4-dimethylmaleimide, characterized in that 3,4-dimethylmaleimide is reacted with dichlorofluoromethanesulphenyl chloride in solution, in the presence of an acid-binding agent.
3. Microbicidal agent containing N-(dichlorofluoromethylthio) -3,4-dimethylmaleimide as the active compound.
4. Microbicidal agent according to Claim 3, containing 1 to 95 % by weight of the active compound.
5. Use of microbicidal agents according to Claim 3, for preserving industrial materials from microbial change and destruction.
6. Use according to Claim 5, characterized in that 0.001 to 5 % by weight of the active compound is used, based on the material to be preserved.

## Revendications

1. Le N-(dichlorofluorométhylthio)-3,4-diméthylmaléimide.
2. Procédé de préparation du N-(dichlorofluorométhylthio) -3,4-diméthylmaléimide, caractérisé en ce que l'on fait réagir le 3,4-diméthylmaléimide avecle chlorure de dichlorofluorométhanesulfényl en présence d'un capteur d'acide en solution.
3. Produit microbicide contenant, en tant que substance active, le N-(dichlorofluorométhylthio)-3,4-diméthylmaléimide.
4. Produit microbicide selon la revendication 3, contenant 1 à 95 % en poids de la substance active.
5. Utilisation de produits microbicides selon la revendication 3 pour la protection de matériaux industriels contre les altérations et la destruction par les microbes.
6. Utilisation selon la revendication 5, caractérisée en ce que l'on utilise de 0,001 à 5 % en poids de la substance active, par rapport au materiau à protéger.